# EUROPÄISCHE PATENTSCHRIFT

(11) **EP 3 684 908 B1**
(45) Veröffentlichungstag und Bekanntmachung des Hinweises auf die Patenterteilung: **14.07.2021**
(21) Anmeldenummer: 19710580.2
(22) Anmeldetag: 04.03.2019
(51) Int. Cl.: C12M 1/00, C12M 1/06, C12M 1/26, C12M 1/34, G01N 1/14, G01N 1/20

(54) **KONZENTRATIONS-MESSVORRICHTUNG FÜR EINEN BEHÄLTER MIT IM WESENTLICHEN FLÜSSIGEM BEHÄLTERINHALT**
CONCENTRATION MEASURING DEVICE FOR A CONTAINER WITH SUBSTANTIALLY LIQUID CONTAINER CONTENT
DISPOSITIF DE MESURE DE CONCENTRATION POUR UN RÉCIPIENT POURVU D'UN CONTENU ESSENTIELLEMENT LIQUIDE

(30) Priorität: 06.03.2018 AT 501822018
(43) Veröffentlichungstag der Anmeldung: 29.07.2020
(73) Patentinhaber: Zeta GmbH, 8501 Lieboch (AT)
(72) Erfinder: MAISCHBERGER, Thomas, 9470 St. Paul im Lavantal (AT); KRAINER, Florian, 8010 Graz (AT)
(74) Vertreter: Röggla, Harald
(86) Internationale Anmeldenummer: PCT/AT2019/060069
(87) Internationale Veröffentlichungsnummer: WO 2019/169416

(56) Entgegenhaltungen:
- EP-A2- 0 184 441
- DE-A1- 3 516 080
- DE-A1- 19 848 542
- DE-A1-102009 037 345
- FR-A1- 2 617 286
- US-A- 5 834 657

## Beschreibung

Messvorrichtung für einen, einen Prozessanschluss aufweisenden, Behälter zur Bestimmung der Konzentration eines Inhaltsstoffes in einem im Wesentlich flüssigen Behälterinhalt, mit einem Konzentrationssensor.

Des Weiteren betrifft die Erfindung ein Messverfahren zur Bestimmung der Konzentration eines Inhaltsstoffs in einem im Wesentlich flüssigen Behälterinhalt eines Behälters.

Behälter zur Aufnahme von einem im Wesentlichen flüssigen Behälterinhalt mit einem Prozessanschluss werden in vielen Industriebereichen, wie beispielsweise in der Lebensmittelindustrie oder der biopharmazeutischen Industrie, eingesetzt. Oftmals findet in diesen ein biologischer Prozess statt, wobei in diesem Fall derartige Behälter als Bioreaktoren bezeichnet werden. In Bioreaktoren sollen möglichst optimale Bedingungen für das Wachstum und die Produktbildung von darin kultivierten Mikroorganismen sowie pflanzlichen, tierischen, insekten oder humanen Zellen bereitgestellt werden. Diese Bedingungen umfassen eine optimale Temperatur, pH Milieu, Osmolarität, Nährstoffangebot (Substrat und Sauerstoff), der Austrag von Metaboliten und CO₂ und lokaler Wärmequellen sowie eine geringe Tendenz zur Schichtungsbildung durch zu hohe Dichteunterschiede (beispielsweise Sedimentation). Diese homogenen Bedingungen werden in Bioreaktoren durch eine geeignete Geometrie des Behälters, durch darin vorhandene Einbauten und Rührwerke mit Rührorganen zur Durchmischung des Behälterinhalts, sowie durch optimale Prozessparameter, wie beispielsweise einer Drehzahl des Rührwerks, optimale Begasungsraten sowie optimalem Gasaustrag und Nährstoffzugabe erzielt. Eine der wichtigsten Kenngrößen für die Beschreibung der Leistungsfähigkeit und die Homogenität beziehungsweise der Mischgüte eines Bioreaktors ist der gas/flüssig-Stofftransport, da vor allem bei aeroben Prozessen Sauerstoff meist das limitierende Substrat für das Wachstum der Mikroorganismen ist. Auch in Behältern mit im Wesentlich flüssigen Behälterinhalt in anderen Industriebereichen ist die Konzentration verschiedener Inhaltsstoffe des Behälterinhalts von Interesse.

Sauerstoff ist einer der wichtigsten Substrate in einem Bioprozess. Für eine Charakterisierung eines in einem Bioreaktor ablaufenden Prozess dient unter anderem eine rechnerische Bestimmung des Stofftransportes aus der Gasphase in die Flüssigphase mittels verschiedener Rechenansätze. Diese berücksichtigen eine Rühraktivität des Rührwerks und eine hierdurch generierte Turbulenz des Behälterinhalts, eine Behältergeometrie und eine damit verbundene Gasblasengröße sowie Steighöhen der Gasblasen, einen Gaseintrag und ein Mediensystem im Bioreaktor.

Messungen zur Bestimmung der Konzentration von Inhaltsstoffen des Behälterinhalts von Bioreaktoren werden gemäß dem Stand der Technik über eine örtliche Messung im Bioreaktor durchgeführt. Verschiedene, am Markt verfügbare Messsysteme sind beispielsweise in der Lage, den in der Flüssigphase des Behälterinhalts gelösten Sauerstoffgehalt zu bestimmen. Es sind ebenfalls Messsysteme bekannt, welche eine Gasbilanzierung mittels Bestimmung des Gaseintrags und des Gasaustrags durchführen.

In Bioreaktoren mit großem Volumen besteht vor allem das Problem der Inhomogenität des Behälterinhalts. Mikroorganismen reagieren meist sehr sensibel auf geringe Schwankungen sowohl von Temperatur, als auch von Nährstoff-, Sauerstoff- und Metabolitkonzentrationen. Diese Inhomogenitäten, Konzentrations-, Temperatur und Sauerstoffgehaltgradienten in großen Bioreaktoren entstehen unter anderem durch Begasungseinheiten, die Geometrie der Substratzugabe, die Prozessparameter und durch die Rührorgane des Rührwerks. Durch das Zusammenspiel der einzelnen Gegebenheiten entstehen in dem Bioreaktor Zonen mit Nährstoffüberangebot und Gebiete mit stark reduziertem Sauerstoffgehalt, meist im oberen Bereich und in Bodennähe. Insbesondere die anaeroben Zonen senken nicht nur die Produktivität, sondern fördern eine irreversible Produktion unerwünschter Nebenprodukte.

Messvorrichtungen gemäß dem Stand der Technik bestimmen Konzentrationen von Inhaltsstoffen in dem Behälter über Standardsensoren an in den Behälter eingeschweißten Stutzen. Die DE 11 2004 002 636 T5 offenbart beispielsweise einen Bioreaktor mit einem an einer Behälterwand angeordneten Sauerstoffsensor. Derartige Messvorrichtungen gemäß dem Stand der Technik weisen den Nachteil auf, dass sie keine Möglichkeit bieten Informationen über die Verteilung von Sauerstoff im Behälterinhalt mit einer befriedigenden Ortsauflösung zu generieren. Besonders nachteilig ist, dass alle am Markt befindlichen Messmethoden zur Bestimmung des Gasgehaltes und des Gastransports die Gemeinsamkeit aufweisen, das Messungen im Innenraum des Bioreaktors beziehungsweise des Behälters immer eine Punktaufnahme darstellen, deren Position bereits bei der Planung des Prozesses sowie dem Design des Behälters genau festgelegt sein muss. Eine Messung mittels Eingangs- und Ausgangswerten ergibt hingegen nur ein gemitteltes Bild über das Gesamtvolumen des Behälters und erlaubt keine Rückschlüsse auf lokale Verteilungen und daraus resultierende Inhomogenitäten.

EP 0 184 441 offenbart einen Bioreaktor mit einer an einen Prozessanschluss angeschlossenen Messvorrichtung. Die Messvorrichtung verfügt über einen nicht im Reaktor befindlichen Konzentrationssensor, einer Entnahmeleitung, einer Pumpe und einem Filterelement.

Am Markt befindliche Messvorrichtungen umfassen Konzentrationssensoren mit unterschiedlichen Ansprechzeiten. Konzentrationssensoren mit längerer Ansprechzeit liefert im Allgemeinen genauere Werte im oberen Messbereich, nahe einer maximalen Sättigung, wobei ein Konzentrationssensor mit kürzerer Ansprechzeit meist genauer im Messbereich niedriger Konzentrationswerte arbeitet. Alle am Markt befindlichen Messvorrichtungen zur Bestimmung der Konzentration von Inhaltsstoffen des Behälterinhaltsweisen jedoch den Nachteil auf, dass am Konzentrationssensor vorbeiströmende Gasblasen den jeweiligen Messwert sehr stark beeinflussen und zu einer starken Schwankung des Messwerts führen. Hierdurch ist es erforderlich Konzentrationssensoren mit höheren Reaktionszeiten von bis zu einer Minute einzusetzen um diesen Effekt abzumindern. Hierdurch ergibt sich allerdings eine Messwertverschiebung in Richtung höherer Konzentrationswerte, da durch die Trägheit des Konzentrationssensors der aktuelle Messwert gemittelt wird. Die Diffusion aus einer Gasblase über eine Membran des Konzentrationssensors in eine Messkammer des Konzentrationssensors verläuft zudem um vieles rascher als eine Diffusion aus der Flüssigphase. Die meisten Messvorrichtungen wenden darüber hinaus eine mathematische Mittelung der Messwerte an, um diese Messwertschwankungen weiter abzuschwächen. Dennoch beeinflussen Gasblasen, welche an den Konzentrationssensoren vorbeiströmen die gemittelten Messwerte in einem beträchtlichen Ausmaß.

Es ist die Aufgabe der vorliegenden Erfindung eine Messvorrichtung zur Bestimmung der Konzentration eines Inhaltsstoffes in einem im Wesentlich flüssigen Behälterinhalt zu bilden, welche die Nachteile des Standes der Technik vermeidet.

Erfindungsgemäß wird die vorliegende Aufgabe dadurch gelöst, dass die Messvorrichtung einen Filter, eine Entnahmeleitung und eine Pumpe umfasst, wobei der Filter in dem Behälter und der Konzentrationssensor außerhalb des Behälters angeordnet ist, und die Entnahmeleitung ausgehend von dem Filter, durch den Prozessanschluss, zu dem Konzentrationssensor verläuft, wobei der Konzentrationssensor mit der Pumpe verbunden ist, und die Pumpe den Behälterinhalt aus dem Behälter durch den Filter über die Entnahmeleitung zu dem Konzentrationssensor befördert, wobei die Entnahmeleitung zumindest eine, zwischen dem Filter und dem Prozessanschluss angeordnete Krümmung aufweist.

Die erfindungsgemäße Messvorrichtung ist in einen Behälter mit einem Prozessanschluss und einem Rührwerkzeug integrierbar.

Die vorliegende Aufgabe der Erfindung wird des Weiteren durch ein Messverfahren zur Bestimmung der Konzentration eines Inhaltsstoffs in einem im Wesentlich flüssigen Behälterinhalt eines Behälters mit einer erfindungsgemäßen Messvorrichtung gelöst, gekennzeichnet durch die Schritte:
a) Befördern von Behälterinhalt aus dem Behälter durch den Filter über die Entnahmeleitung zu dem Konzentrationssensor durch die Pumpe;
b) Bestimmen der Konzentration des Inhaltsstoffs in dem zu dem Konzentrationssensor beförderten Behälterinhalt durch den Konzentrationssensor.

Die erfindungsgemäße Messvorrichtung für einen, einen Prozessanschluss aufweisenden, Behälter zur Bestimmung des Sauerstoffgehalts von einem im Wesentlichen flüssigen Behälterinhalt umfasst einen Konzentrationssensor, einen Filter, eine Entnahmeleitung und eine Pumpe. Der Filter ist in dem Behälter, und der Konzentrationssensor ist außerhalb des Behälters angeordnet. Die Entnahmeleitung verläuft ausgehend von dem Filter durch den Prozessanschluss des Behälters zu dem Konzentrationssensor. Der Konzentrationssensor ist mit der Pumpe verbunden. Die Pumpe befördert den Behälterinhalt aus dem Behälter durch den Filter über die Entnahmeleitung zu dem Konzentrationssensor. Anschließend bestimmt der Konzentrationssensor den Sauerstoffgehalt des zu dem Konzentrationssensor beförderten Behälterinhalts. Die erfindungsgemäße Messvorrichtung entnimmt zur Bestimmung des Sauerstoffgehalts des Behälterinhalts eine geringe Menge an Behälterinhalt mittels der Entnahmeleitung aus dem Behälter. Hierbei wird der Behälterinhalt von der Pumpe durch den Filter gesaugt, wobei an dem Filter gasförmiger Sauerstoff, in Form von Gasblasen, abgeschieden wird. Hierdurch wird der Vorteil erreicht, dass die Gasblasen keinen Einfluss auf den Messvorgang haben, und das Messergebnis nicht beeinflussen können. Besonders vorteilhaft ist, dass hierdurch keine mathematische Mittelung der gemessenen Werte notwendig ist, sofern diese zur Glättung schwankender Messwerte im Stand der Technik in einem Konkreten Anwendungsfall herangezogen wurde. Des Weiteren wird hierdurch der Vorteil erreicht, dass verschiedene Konzentrationssensoren verwendet werden können, welche sich, beispielsweise aufgrund ihrer geringen mechanischen Belastbarkeit, nicht für einen Einsatz im Behälterinneren eignen oder zu große Messfehler bei der Gegenwart von Gasblasen im Behälterinhalt produzieren würden. Dies ermöglicht den Einsatz von Konzentrationssensoren mit Reaktionszeiten von weniger als einer Sekunde, wodurch vorteilhafterweise eine hohe Zeitauflösung der Sauerstoffgehaltmessung bereitgestellt werden kann. Ein weiterer Vorteil der erfindungsgemäßen Messvorrichtung besteht darin, sowohl neue, als auch bestehende Behälter, beziehungsweise Bioreaktoren, mit der steril arbeitenden, reinigbaren und sterililsierbaren erfindungsgemäßen Messvorrichtung auszurüsten.

Vorteilhafte Ausgestaltungen der erfindungsgemäßen Vorrichtung und des erfindungsgemäßen Verfahrens, sowie alternativer Ausführungsvarianten werden in weiterer Folge anhand der Figuren näher erläutert.
Figur 1 zeigt einen Behälter mit einer erfindungsgemäßen Messvorrichtung in einer schematischen Querschnittsansicht.
Figur 2 zeigt die erfindungsgemäße Messvorrichtung aus Figur 1 mit einer Durchführung durch einen Prozessanschluss des Behälters.

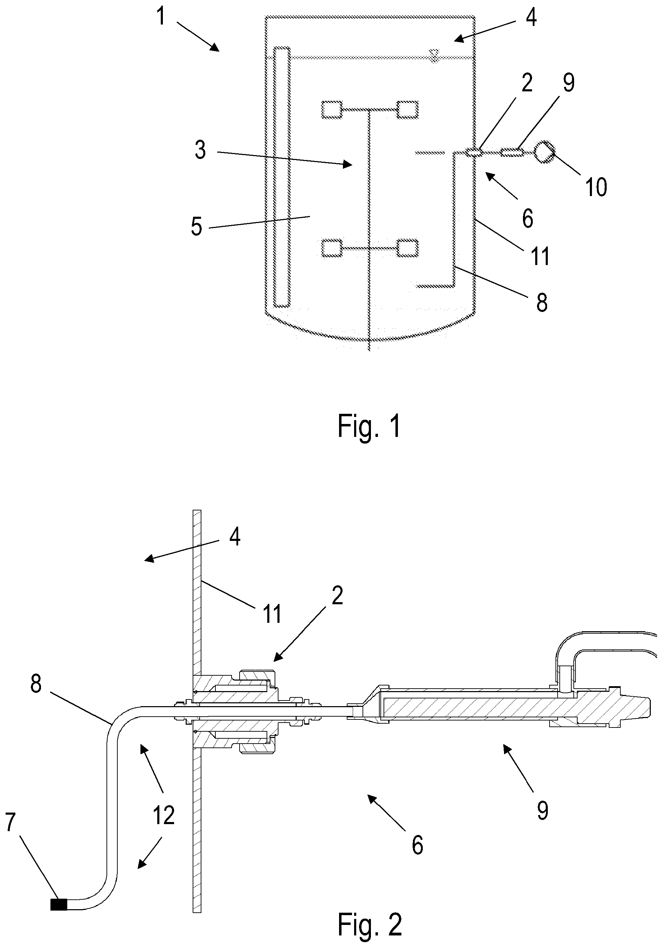

Figur 1 zeigt einen Behälter 1 mit einem Prozessanschluss 2 und einem Rührwerkzeug 3, welches in einem Innenraum 4 des Behälters 1 angeordnet ist, in einer schematischen Ansicht. In dem Innenraum 4 des Behälters 3 ist ein im Wesentlichen flüssiger Behälterinhalt 5 aufgenommen. Das Rührwerkzeug 3 wird von außerhalb des Behälters 1 angetrieben und führt eine Rotationsbewegung aus, wodurch eine Durchmischung des Behälterinhalts 5 bewirkt wird. Der Behälter 1 weist des Weiteren eine Messvorrichtung 6 zur Bestimmung der Konzentration eines Inhaltsstoffes des Behälterinhalts 5 auf. Die Messvorrichtung 6 umfasst einen Filter 7, welcher in Figur 2 dargestellt ist, eine Entnahmeleitung 8, einen Konzentrationssensor 9 sowie eine Pumpe 10. Der Filter 7 ist in dem Innenraum 4 des Behälters 1 angeordnet und der Konzentrationssensor 9 ist außerhalb des Behälters 1 angeordnet. Die Entnahmeleitung 8 verläuft ausgehend von dem Filter 7, durch den Prozessanschluss 2 des Behälters 1 zu dem Konzentrationssensor 9. Die Entnahmeleitung 8 führt durch den Prozessanschluss 2 hindurch und durchtritt in diesem eine Wand 11 des Behälters 1. Die Entnahmeleitung 8 ist in dem Prozessanschluss 2 mittels einer Klemmverschraubung abgedichtet. Der Konzentrationssensor 9 ist mit der Pumpe 10 verbunden, wobei die Pumpe 10 im Zuge eines Messvorgangs den Behälterinhalt 5 aus dem Innenraum 4 des Behälters 1 durch den Filter 7 und die Entnahmeleitung 8 zu dem Konzentrationssensor 9 befördert. Der Konzentrationssensor 9 bestimmt die Konzentration des Inhaltsstoffes des Behälterinhalts 5. Ein in einem gasförmigen Zustand vorliegender Inhaltsstoff, wie beispielsweise Sauerstoff oder CO₂, in Form von Gasblasen, welche sich im Behälterinhalt 5 befinden, wird an dem Filter 7 im Innenraum 4 des Behälters 1 abgeschieden. Hierdurch wird der Vorteil erreicht, dass kein gasförmiger Inhaltsstoff zu dem Konzentrationssensor 9 außerhalb des Behälters 1 über die Entnahmeleitung 8 gelangt. Besonders vorteilhaft ist, dass hierdurch eine höhere Messgenauigkeit im Vergleich zu Messvorrichtungen gemäß dem Stand der Technik erreicht wird. Ein weiterer Vorteil besteht darin, dass hierdurch ermöglicht wird, Konzentrationssensoren 9 mit einer geringen Ansprechzeit, beispielsweise unter einer Sekunde, in der erfindungsgemäßen Messvorrichtung 6 einzusetzen, wodurch geringe Konzentrationsunterschiede und - veränderungen mit der erfindungsgemäßen Messvorrichtung 6 nachweisbar sind. Des Weiteren erfordert die erfindungsgemäße Messvorrichtung 6 keine mathematische Glättung der Messwerte. Die Pumpe 10 ist gemäß der bevorzugten Ausführungsform der Messvorrichtung 6 als Schlauchquetschpumpe ausgebildet. Hierdurch wird der Vorteil erreicht, dass der Behälterinhalt 5 in der Entnahmeleitung 8 kontinuierlich und im Wesentlichen ohne Druckschwankungen zu dem Konzentrationssensor 9 befördert wird.

Figur 2 zeigt die in Figur 1 dargestellte Messvorrichtung 6 in einer Schnittansicht mit dem Filter 7, der Entnahmeleitung 8 und dem Konzentrationssensor 9 in einer bevorzugten Ausführungsform. Die Pumpe 10 ist in Figur 2 nicht dargestellt. Des Weiteren ist der Prozessanschluss 2 des Behälters 1 mit der, durch diesen hindurchgeführten, Entnahmeleitung 8, sowie ein Abschnitt der Wand 11 des Behälters 1 dargestellt. Die Entnahmeleitung 8 weist zwei Krümmungen 12 auf, welche zwischen dem Filter 7 und dem Prozessanschluss 2 angeordnet sind. In einer alternativen Ausführungsvariante der Messvorrichtung 6 weist die Entnahmeleitung 8 zumindest eine Krümmung 12 auf. Durch die Krümmung 12 wird der Vorteil erreicht, dass die Entnahmeleitung 8 an die Geometrie des Innenraums 4 des Behälters 1 und an die Geometrie der in Figur 1 beispielshaft dargestellten Rührwerkzeuge 3 anpassbar ist. Besonders vorteilhaft ist, dass hierdurch unterschiedliche Entnahmepositionen für Behälterinhalt 5, welcher in Figur 2 nicht dargestellt ist, im Innenraum 4 des Behälters 1 bei Verwendung des standardisierten Prozessanschlusses 2 erreicht werden können. Ein weiterer Vorteil besteht darin, dass im Gegensatz zu Messvorrichtungen des Standes der Technik mittels nur eines Konzentrationssensors 9 die Konzentration des Inhaltsstoffes im Behälterinhalt an unterschiedlichen frei wählbaren Messpositionen im Innenraum 4 des Behälters 1 bestimmt werden kann.

Die Entnahmeleitung 8 ist gemäß der bevorzugten Ausführungsform der Messvorrichtung 6 drehbar in dem Prozessanschluss 2 gelagert. Hierdurch wird der Vorteil erreicht, dass in Kombination mit der gekrümmten Entnahmeleitung 8 eine Vielzahl an unterschiedlichen Entnahmepositionen erreicht werden kann. Besonders vorteilhaft ist, dass hierdurch mittels Drehung der Entnahmeleitung 8 beispielsweise ein Konzentrationsverlauf im Behälterinhalt entlang einer Höhe des Behälters 1 mit der erfindungsgemäßen Messvorrichtung 6 erfasst werden kann. Gemäß der bevorzugten Ausführungsform der Messvorrichtung 6 ist die Entnahmeleitung 8 des Weiteren in dem Prozessanschluss 2 verschiebbar gelagert. Hierdurch wird der Vorteil erreicht, dass die Entnahmeposition ebenfalls in radialer Richtung des Behälters 1 beziehungsweise in einer Behältertiefe variiert werden kann. Hierdurch ergeben sich weitere Möglichkeiten für die Erstellung von Konzentrationsverläufen. Durch entsprechende Anpassung der Krümmung 12 oder der Krümmungen 12 der Entnahmeleitung 8 werden Kollisionen der Entnahmeleitung 8 mit den Rührwerkzeugen 3 im Behälterinneren 4 vermieden. Hierdurch wird der Vorteil erreicht, dass auch während des Betriebes des oder der Rührwerkzeuge 3 Messungen mit der erfindungsgemäßen Messvorrichtung 6 durchgeführt werden können.

Der Konzentrationssensor 9 ist gemäß der bevorzugten Ausführungsform der erfindungsgemäßen Messvorrichtung 6 ein Durchflusssensor. Hierdurch wird der Vorteil erreicht, dass eine kontinuierliche Messung der Konzentration bereitgestellt werden kann, und mathematische Interpolationsrechnungen bei der Erstellung eines Konzentrationsprofils des Inhaltsstoffes des Behälterinhalts 5 aus einzelnen Messpunkten vermieden werden. Vorzugsweise weist der Konzentrationssensor 9 eine Reaktionszeit von unter einer Sekunde auf. Hierdurch wird der Vorteil erreicht, dass auch geringe Schwankungen der Konzentration zuverlässig detektiert werden. Der Durchflusssensor versetzt den Behälterinhalt 5 in eine turbulente Strömung. Die turbulente Strömung wird beispielweise mit einer Reihe an Vorsprüngen in einem Strömungsgang des Durchflusssensor erzeugt. Weitere Möglichkeiten zur Erzeugung einer turbulenten Strömung ergeben sich für den Fachmann aus diesem beispielhaften Verweis. Hierdurch wird der Vorteil erreicht, dass eine Trennung von im Behälterinhalt 5 gelösten gasförmigen Inhaltsstoffen aus der flüssigen Phase in die gasförmige Phase im Durchflusssensor vermieden wird. Die Messvorrichtung 6 weist zudem gemäß der bevorzugten Ausführungsform eine Rückführleitung auf, welche in den Figuren nicht dargestellt ist. Die Rückführleitung ist mit der Pumpe 10 und dem Behälter 1 verbunden. Die Integration der Rückführleitung weist den Vorteil auf, dass aus dem Behälter 1 entnommener Behälterinhalt 5 wieder in den Behälter 1 rückgeführt wird. Besonders vorteilhaft ist, dass eine Messung der Konzentration mit der erfindungsgemäßen Messvorrichtung 6 somit zu keinem Schwund des Behälterinhalts 5 führt.

Der Filter 7 ist gemäß der bevorzugten Ausführungsvariante der erfindungsgemäßen Messvorrichtung 6 ein Gewebesieb. Insbesondere ist der Filter 7 ein metallisches Gewebesieb. Hierdurch wird der Vorteil erreicht, dass die Oberfläche des Filters 7 gegenüber Flüssigkeiten einen geringen Strömungswiderstand aufweist und gleichzeitig für Gasblasen nicht passierbar ist. Das metallische Gewebesieb besteht vorzugsweise aus einem Edelstahlmaterial, mit einer Materialgüte, welche jener des Behälters 1 entspricht. Zudem ist das Gewebesieb reinigbar, sterilisierbar, beziehungsweise autoklavierbar.

Gemäß einer Ausführungsvariante der erfindungsgemäßen Messvorrichtung 6 ist der Konzentrationssensor 9 ein Gasgehaltssensor, wie ein Sauerstoffgehaltssensor oder ein CO₂-Gehaltssensor, oder ein pH-Wert Sensor. Hierdurch wird der Vorteil erreicht, dass verschiedene Parameter des Behälterinhalts mit der erfindungsgemäßen Vorrichtung erfassbar sind.

Die erfindungsgemäße Messvorrichtung 6 ist direkt in den, wie unter Figur 1 beschriebenen, Behälter 1, integrierbar, welcher den Prozessanschluss 2 und das Rührwerkzeug 3 aufweist.

Der Prozessanschluss 2 ist vorzugsweise ein TC Anschluss oder eine Ingold-Anschluss. Weitere Prozessanschlüsse 2 ergeben sich für den Fachmann aus diesem beispielhaften Verweis. Hierdurch wird der Vorteil erreicht, dass die erfindungsgemäße Messvorrichtung 6 in Behältern 1 mit am Markt üblichen Prozessanschlüssen 2 integrierbar ist.

Mit der erfindungsgemäßen Messvorrichtung 6 wird ein Verfahren zur Bestimmung der Konzentration des Inhaltsstoffes in dem im Wesentlichen flüssigen Behälterinhalt 5 eines mit der erfindungsgemäßen Messvorrichtung 6 ausgestatteten Behälters 1 durchgeführt. Zur Bestimmung der Konzentration wird in einem ersten Verfahrensschritt mittels der Pumpe 10 Behälterinhalt 5 aus dem Behälter 1 durch den Filter 7 über die Entnahmeleitung 8 zu dem Konzentrationssensor 9 befördert. Im Anschluss wird die Konzentration des Inhaltsstoffes in dem zu dem Konzentrationssensor 9 beförderten Behälterinhalt 5 mittels des Konzentrationssensors 9 bestimmt. Hierdurch wird der Vorteil erreicht, dass eventuell im Behälterinhalt 5 vorhandene Gasblasen an dem Filter 7 abgeschieden werden, und nicht zum Konzentrationssensor 9 befördert werden.

Gemäß der bevorzugten Ausführungsform des erfindungsgemäßen Verfahrens wird während der Behälterinhalt 5 aus dem Behälter 1 durch den Filter 7 über die Entnahmeleitung 8 zu dem Konzentrationssensor 9 befördert wird die Entnahmeleitung 8 in dem Prozessanschluss 2 gedreht und/oder verschoben. Hierdurch wird der Vorteil erreicht, dass ein Konzentrationsprofil des Inhaltsstoffes in dem Behälterinhalt 5 sowohl über verschiedene Höhenschichten des Behälters 1 und/oder in Richtung eines Behälterzentrums erstellt wird.

Die erfindungsgemäße Vorrichtung und das erfindungsgemäße Verfahren zur Bestimmung der Konzentration des Inhaltsstoffes in dem im Wesentlich flüssigen Behälterinhalt 5 eignet sich gleichermaßen zur Bestimmung von weiteren physikalischen und/oder chemischen Parametern des Behälterinhalts 5. Hierbei wird der Konzentrationssensor 9 durch einen für den zu bestimmenden Parameter sensitiven Sensor ersetzt.

Besonders vorteilhaft ist es die Geometrie und die Verschieb- und Verdrehbarkeit der Entnahmeleitung 8 so auszubilden, dass eine Kollision mit dem sich drehenden Rührwerk 3 zuverlässig verhindert wird. Die die Messung durchführende Person muss folglich nicht fürchten den Filter 7 oder das Rührwerk 3 zu beschädigen, wenn während das Rührwerk 3 den flüssigen Behälterinhalt 5 durchmischt der Sauerstoffgehalt gemessen werden soll.

Es kann erwähnt werden, dass der flüssige Behälterinhalt von dünnflüssig bis zäh- und breiig ausgebildet sein kann.

## Patentansprüche

1. Messvorrichtung (6) für einen, einen Prozessanschluss (2) aufweisenden, Behälter (1) zur Bestimmung der Konzentration eines Inhaltsstoffes in einem im Wesentlich flüssigen Behälterinhalt (5), mit einem Konzentrationssensor (9) **dadurch gekennzeichnet, dass** die Messvorrichtung (6) einen Filter (7), eine Entnahmeleitung (8) und eine Pumpe (10) umfasst, wobei der Filter (7) in dem Behälter (1) und der Konzentrationssensor (9) außerhalb des Behälters (1) angeordnet ist, und die Entnahmeleitung (8) ausgehend von dem Filter (7), durch den Prozessanschluss (2), zu dem Konzentrationssensor (9) verläuft, wobei der Konzentrationssensor (9) mit der Pumpe (10) verbunden ist, und die Pumpe (10) den Behälterinhalt (5) aus dem Behälter (1) durch den Filter (7) über die Entnahmeleitung (8) zu dem Konzentrationssensor (9) befördert, wobei die Entnahmeleitung (8) zumindest eine, zwischen dem Filter (7) und dem Prozessanschluss (2) angeordnete Krümmung (12) aufweist.

2. Messvorrichtung (6) gemäß Anspruch 1, **dadurch gekennzeichnet, dass** die Entnahmeleitung (8) in dem Prozessanschluss (2) drehbar gelagert ist.

3. Messvorrichtung (6) gemäß einem der Ansprüche 1 bis 2, **dadurch gekennzeichnet, dass** die Entnahmeleitung (8) in dem Prozessanschluss (2) verschiebbar gelagert ist.

4. Messvorrichtung (6) gemäß einem der Ansprüche 1 bis 3, **dadurch gekennzeichnet, dass** der Konzentrationssensor (9) ein Durchflusssensor ist.

5. Messvorrichtung (6) gemäß Anspruch 4, **dadurch gekennzeichnet, dass** der Durchflusssensor dazu ausgebildet ist den Behälterinhalt (5) in eine turbulente Strömung zu versetzen.

6. Messvorrichtung (6) gemäß einem der Ansprüche 1 bis 5, **dadurch gekennzeichnet, dass** die Pumpe (10) eine Schlauchquetschpumpe ist.

7. Messvorrichtung (6) gemäß einem der Ansprüche 1 bis 6, **dadurch gekennzeichnet, dass** die Messvorrichtung (6) eine Rückführleitung aufweist, wobei die Rückführleitung mit der Pumpe (10) und dem Behälter (1) verbunden ist.

8. Messvorrichtung (6) gemäß einem der Ansprüche 1 bis 7, **dadurch gekennzeichnet, dass** der Filter (7) ein Gewebesieb ist.

9. Messvorrichtung (6) gemäß Anspruch 8, **dadurch gekennzeichnet, dass** das Gewebesieb ein metallisches Gewebesieb ist.

10. Messvorrichtung (6) gemäß einem der Ansprüche 1 bis 9, **dadurch gekennzeichnet, dass** der Konzentrationssensor (9) eine Reaktionszeit von unter einer Sekunde aufweist.

11. Messvorrichtung (6) gemäß einem der Ansprüche 1 bis 10, **dadurch gekennzeichnet, dass** der Konzentrationssensor (9) ein Gasgehaltssensor oder ein pH-Wert Sensor ist.

12. Messvorrichtung (6) gemäß einem der Ansprüche 1 bis 10, **dadurch gekennzeichnet, dass** der Konzentrationssensor (9) ein Sauerstoffgehaltssensor oder ein CO₂-Gehaltssensor ist.

13. Behälter (1) mit einer Messvorrichtung (6) gemäß einem der Ansprüche 1 bis 10, wobei der Behälter (1) einen Prozessanschluss (2) und ein Rührwerkzeug (3) aufweist.

14. Behälter (1) gemäß Anspruch 13, **dadurch gekennzeichnet, dass** der Prozessanschluss (2) ein TC-Anschluss oder ein Ingold-Anschluss ist.

15. Messverfahren zur Bestimmung der Konzentration eines Inhaltsstoffs in einem im Wesentlich flüssigen Behälterinhalt (5) eines Behälters (1) mit einer Messvorrichtung (6) gemäß einem der Ansprüche 1 bis 12, **gekennzeichnet durch** die Schritte:
a) Befördern von Behälterinhalt (5) aus dem Behälter (1) durch den Filter (7) über die Entnahmeleitung (8) zu dem Konzentrationssensor (9) durch die Pumpe (10);
b) Bestimmen der Konzentration des Inhaltsstoffs in dem zu dem Konzentrationssensor (9) beförderten Behälterinhalt (5) durch den Konzentrationssensor (9).

16. Messverfahren gemäß Anspruch 15, **gekennzeichnet durch** die Schritte:
c) Drehen der Entnahmeleitung (8) in dem Prozessanschluss (2) während des Schrittes
a) und/oder
d) Verschieben der Entnahmeleitung (8) in dem Prozessanschluss (2) während des Schrittes a).

## Claims

1. A measuring device (6) for a container (1) having a process fitting (2) for determining the concentration of a constituent in a substantially liquid container content (1) using a concentration sensor (9), **characterized in that** the measuring device (6) comprises a filter (7), a retrieval line (8) and a pump (10), wherein the filter (7) is arranged within the container (1) and the concentration sensor (9) external to the container (1), and that the retrieval line (8) extend starting from the filter (7) through the process fitting (2) to the concentration sensor (9), wherein the concentration sensor (9) is connected to the pump (10) and the pump (10) conveys the container content (5) from the container (1) through the filter (7) via the retrieval line (8) to the concentration sensor (9), wherein the retrieval line (8) has at least one curvature (12) arranged between the filter (7) and the process fitting (2).

2. A measuring device (6) according to claim 1, **characterized in that** the retrieval line (8) is rotatably mounted within the process fitting (2).

3. A measuring device (6) according to any of claims 1 to 2, **characterized in that** the retrieval line (8) is slidably mounted within the process fitting (2).

4. A measuring device (6) according to any of claims 1 to 3, **characterized in that** the concentration sensor (9) is a flow sensor.

5. A measuring device (6) according to claim 4, **characterized in that** the flow sensor is configured to put the container content (5) into a turbulent flow.

6. A measuring device (6) according to any of claims 1 to 5, **characterized in that** the pump (10) is a peristaltic pump.

7. A measuring device (6) according to any of claims 1 to 6, **characterized in that** the measuring device (6) has a return line, wherein the return line is connected to the pump (10) and the container (1).

8. A measuring device (6) according to any of claims 1 to 7, **characterized in that** the filter (7) is a fabric sieve.

9. A measuring device (6) according to claim 8, **characterized in that** the fabric sieve is a metallic fabric sieve.

10. A measuring device (6) according to any of claims 1 to 9, **characterized in that** the concentration sensor (9) has a reaction time of less than one second.

11. A measuring device (6) according to any of claims 1 to 10, **characterized in that** the concentration sensor (9) is a gas content sensor or a pH value sensor.

12. A measuring device (6) according to any of claims 1 to 10, **characterized in that** the concentration sensor (9) is an oxygen content sensor or a CO₂ content sensor.

13. A container (1) having a measuring device (6) according to any of claims 1 to 10, wherein the container (1) has a process fitting (2) and a stirring tool (3).

14. A container (1) according to claim 13, **characterized in that** the process fitting (2) is a TC fitting or an Ingold fitting.

15. A measuring method for determining the concentration of a constituent in a substantially liquid container content (5) of a container (1) using a measuring device (6) according to any of claims 1 to 12, **characterized by** the steps of:
a) conveying the container content (5) from the container (1) through the filter (7) via the retrieval line (8) to the concentration sensor (9) by the pump (10);
b) determining the concentration of the constituent in the container content (5) conveyed to the concentration sensor (9) by the concentration sensor (9).

16. A measuring method according to claim 15, **characterized by** the steps of:
c) rotating the retrieval line (8) within the process fitting (2) during the step a) and/or
d) shifting the retrieval line (8) within the process fitting (2) during the step a).

## Revendications

1. Dispositif de mesure (6) pour un récipient (1) doté d'un raccord de processus (2) pour déterminer la concentration d'une matière contenue dans un contenu de récipient (5) essentiellement liquide, avec un capteur de concentration (9), **caractérisé en ce que** ce dispositif de mesure (6) comprend un filtre (7), une conduite de prélèvement (8) et une pompe (10), le filtre (7) étant disposé dans le récipient (1) et le capteur de concentration (9) à l'extérieur du récipient (1), et la conduite de prélèvement (8) courant du filtre (7), via le raccord de processus (2), au capteur de concentration (9), le capteur de concentration (9) étant relié à la pompe (10) et la pompe (10) transportant le contenu de récipient (5) du récipient (1), via le filtre (7), par la conduite de prélèvement (8) au capteur de concentration (9), la conduite de prélèvement (8) présentant au moins une courbure (12) disposée entre le filtre (7) et le raccord de processus (2).

2. Dispositif de mesure (6) selon la revendication 1, **caractérisé en ce que** la conduite de prélèvement (8) est montée de façon pivotante dans le raccord de processus (2).

3. Dispositif de mesure (6) selon l'une des revendications 1 à 2, **caractérisé en ce que** la conduite de prélèvement (8) est montée de façon coulissante dans le raccord de processus (2).

4. Dispositif de mesure (6) selon l'une des revendications 1 à 3, **caractérisé en ce que** le capteur de concentration (9) est un capteur de débit.

5. Dispositif de mesure (6) selon la revendication 4, **caractérisé en ce que** le capteur de débit est conçu pour communiquer au contenu de récipient (5) un écoulement turbulent.

6. Dispositif de mesure (6) selon une des revendications 1 à 5, **caractérisé en ce que** la pompe (10) est une pompe péristaltique.

7. Dispositif de mesure (6) selon l'une des revendications 1 à 6, **caractérisé en ce que** ce dispositif de mesure (6) présente une conduite de retour, cette conduite de retour étant reliée à la pompe (10) et au récipient (1).

8. Dispositif de mesure (6) selon l'une des revendications 1 à 7, **caractérisé en ce que** le filtre (7) est un tamis en tissu.

9. Dispositif de mesure (6) selon la revendication 8, **caractérisé en ce que** le tamis en tissu est un tamis en tissu métallique.

10. Dispositif de mesure (6) selon l'une des revendications 1 à 9, **caractérisé en ce que** le capteur de concentration (9) présente un temps de réaction de moins d'une seconde.

11. Dispositif de mesure (6) selon l'une des revendications 1 à 10, **caractérisé en ce que** le capteur de concentration (9) est un capteur de teneur en gaz ou un capteur de valeur pH.

12. Dispositif de mesure (6) selon l'une des revendications 1 à 10, **caractérisé en ce que** le capteur de concentration (9) est un capteur de teneur en oxygène ou un capteur de teneur en CO₂.

13. Conteneur (1) avec un dispositif de mesure (6) selon l'une des revendications 1 à 10, ce conteneur (1) présentant un raccord de processus (2) et un instrument agitateur (3).

14. Conteneur (1) selon la revendication 13, **caractérisé en ce que** le raccord de processus (2) est un raccord TC ou un raccord Ingold.

15. Procédé de mesure pour déterminer la concentration d'une matière contenue dans un contenu de récipient (5) essentiellement liquide d'un récipient (1) avec un dispositif de mesure (6) selon l'une des revendications 1 à 12, **caractérisé par** les étapes suivantes :
a) transport du contenu de récipient (5) du récipient (1) à travers le filtre (7) par la conduite de prélèvement (8) jusqu'au capteur de concentration (9) au moyen de la pompe (10) et
b) détermination de la concentration de la matière contenue dans le contenu de récipient (5) transporté jusqu'au capteur de concentration (9) par le capteur de concentration (9).

16. Procédé de mesure selon la revendication 15, **caractérisé par** les étapes suivantes :
c) rotation de la conduite de prélèvement (8) dans le raccord de processus (2) pendant l'étape a) et/ou
d) coulissement de la conduite de prélèvement (8) dans le raccord de processus (2) pendant l'étape a).
